# FASCICULE DE BREVET EUROPEEN

(11) **EP 3 621 959 B1**
(45) Date de publication et mention de la délivrance du brevet: **09.06.2021**
(21) Numéro de dépôt: 18726849.5
(22) Date de dépôt: 27.04.2018
(51) Int. Cl.: C07D 403/06, A61K 8/49, C07D 207/10, C07D 207/12, C07D 207/16

(54) **PROCÉDÉ DE SYNTHÈSE D'UN COMPOSÉ N-ACYLÉ SANS UTILISER DE SOLVANT ORGANIQUE NI DE CHLORURE D'ACIDE**
VERFAHREN ZUR SYNTHESE EINER N-ACYLIERTEN VERBINDUNG OHNE VERWENDUNG ORGANISCHER LOESUNGSMITTEL SOWIE SÄURECHLORID
PROCESS FOR THE SYNTHESIS OF A N-ACYLATED COMPOUND WITHOUT USING AN ORGANIC SOLVENT AND ACID CHLORIDE

(30) Priorité: 12.05.2017 FR 1754188
(43) Date de publication de la demande: 18.03.2020
(73) Titulaire: Société d'Exploitation de Produits pour les Industries Chimiques SEPPIC, 75321 Paris cedex 07 (FR)
(72) Inventeur: DACOSTA, Georges, 81710 Saix (FR); GUILBOT, Jérôme, 81100 Castres (FR); PUJOL, Eric, 75321 Paris Cedex 07 (FR); POMMERY, Virginie, 81500 Garrigues (FR); SAUNAL, Fabrice, 75321 Paris Cedex 07 (FR)
(74) Mandataire: Air Liquide
(86) Numéro de dépôt international: PCT/FR2018/051072
(87) Numéro de publication internationale: WO 2018/206878

(56) Documents cités:
- EP-A1- 2 870 958
- EP-A1- 2 889 306
- FR-A1- 3 029 411
- US-A- 3 836 551

## Description

La présente invention concerne un nouveau procédé de préparation de dérivés N-acylés de la proline, n'impliquant par l'utilisation de solvant, ni de chlorures d'acide, et présentant un taux de conversion élevé des matières premières mises en œuvre. La présente invention concerne également l'utilisation des produits acylés obtenus pour préparer des compositions cosmétiques, pharmaceutiques à usage topique ou des compositions de détergence industrielle.

Les dérivés N-acylés d'acides aminés ou également appelés LipoAminoAcides (LAA), sont des agents tensioactifs anioniques, qui sont constitués par une tête polaire provenant d'un résidu d'au moins un acide aminé, ou bien d'un résidu d'(oligo)-peptides ou bien de résidus d'hydrolysats partiels ou totaux de protéines, et par une chaîne hydrocarbonée de nature lipophile, provenant de chlorures d'acides ou d'ester méthyliques, eux-mêmes issus de la filière oléochimique.
Ces dérivés N-acylés d'acides aminés, d'(oligo)-peptides ou bien d'hydrolysats partiels ou totaux de protéines, sont communément utilisés tout d'abord comme ingrédient apportant des propriétés moussantes et nettoyantes pour la préparation de compositions cosmétiques, comme par exemple des gels douche ou des shampoings, ou bien comme ingrédients apportant des propriétés biologiques pour la préparation de compositions cosmétiques destinées à prévenir ou corriger les effets inesthétiques de la peau ; lesdites propriétés biologiques sont par exemple des propriétés antiâges, amincissantes, raffermissantes, dépigmentantes, pro-pigmentantes.

Le procédé de préparation communément mis en œuvre pour la préparation de tels dérivés N-acylés d'acides aminés, d' (oligo)-peptides ou bien d'hydrolysats partiels ou totaux de protéines est connu de l'homme du métier sous le nom de réaction de Schotten Baumann.

Un tel procédé est par exemple divulgué dans les brevets américains US 2,463,779 et US 6,703,517, dans la publication J. Am. Oil Chem. Soc. - 78 (1956) 172, et dans les demandes internationales publiées sous les numéros WO 92/21318 et WO 94/26694.

Ce procédé d'acylation comprend une étape préalable de salification de l'acide aminé, suivie d'une étape d'acylation du sel d'aminoacide avec un chlorure d'acide, puis de l'acidification du sel N-acylé obtenu.

La première étape (voir Schéma 1) consiste à neutraliser l'acide aminé, solubilisé préalablement dans de l'eau ou dans un mélange d'eau et d'un co-solvant organique, par une base minérale, le plus souvent de la soude ou de la potasse aqueuse. Le groupement carboxyle se retrouve alors sous une forme ionisée, permettant ainsi une meilleure solubilité de l'acide aminé dans l'eau. Le pH de cette solution aqueuse se situe entre 9,0 et 12,0, ce qui permet de s'assurer que la fonction amine de l'acide aminé n'est pas protonée.

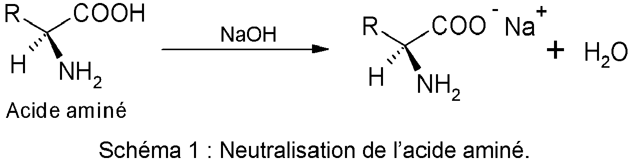

La seconde étape (voir Schéma 2) est l'étape d'acylation à proprement dite. A ce stade, le chlorure d'acide est ajouté progressivement à la solution neutralisée d'acide aminé, à température ambiante. La fonction amine nucléophile attaque le carbone électrophile de la fonction carbonyle. Il en résulte la formation d'une liaison amide entre les deux substrats de départ ainsi que la formation d'acide chlorhydrique. Cet acide est directement neutralisé *in situ* par ajout progressif d'une base minérale (régulation du pH autour de 10,0).

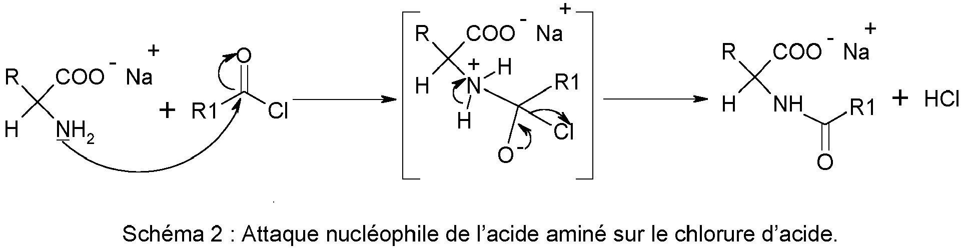

A ce stade, la réaction secondaire d'hydrolyse du chlorure d'acide en savon (voir Schéma 3) est également possible. Elle doit cependant être minimisée de façon à atteindre une conversion satisfaisante de l'acide aminé en son dérivé N-acylé et à les isoler efficacement, car une teneur trop élevée en savon peut induire un déphasage du milieu réactionnel et/ou des problèmes d'odeur ou de toxicité (chaines C8 et C11' par exemple).

Les deux principaux paramètres réactionnels qui permettent de contrôler la formation de savon sont :
i) - La vitesse d'agitation lors de la phase réactionnelle, dont l'optimisation permet une amélioration de la surface de contact du chlorure avec le milieu, et
ii) - l'ajout éventuel, lors de l'étape de solubilisation de l'acide aminé, d'un co-solvant d'acylation, comme par exemple l'acétone, la méthyl éthyl cétone, l'isopropanol, ou des glycols.

Cet ajout de co-solvant permet d'améliorer l'affinité du chlorure d'acide avec le milieu réactionnel. Dans un tel cas, le co-solvant d'acylation doit être choisi judicieusement de façon à éviter ou minimiser la formation de nouveaux produits secondaires issus de la réaction entre ce même co-solvant et le chlorure d'acide, comme par exemple des sous-produits provenant de réactions secondaires d'estérification.

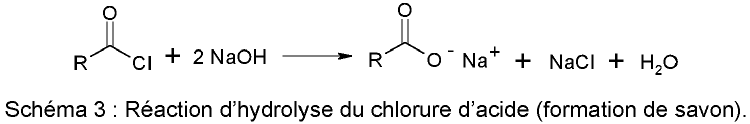

La dernière étape consiste en une étape de finition, de mise en forme du dérivé N-acylé formé, et deux alternatives sont possibles :
i) - La première consiste à régler la valeur du pH du milieu réactionnel obtenu autour de 7. Le dérivé N-acylé est isolé tel quel en solution, sans aucune purification supplémentaire, et comprend les sels d'acylation, les acides aminés non réagis, de l'éventuel co-solvant). Il se présente ainsi sous une forme salifiée, et plus précisément une forme carboxylate, en solution aqueuse et avec une pureté généralement inférieure à 50%.
ii) - La seconde consiste à faire précipiter le dérivé N-acylé en acidifiant le mélange réactionnel à une valeur de pH voisine de 2, puis à réaliser plusieurs opérations de filtration et de lavage, se concluant par un séchage final du milieu obtenu. Cette procédure permet ainsi d'éliminer tous les sels générés au cours de la réaction d'acylation, l'éventuel co-solvant d'acylation et tous les acides aminés non réagis. Dans ce cas, le dérivé N-acylé se présente sous une forme non salifiée, avec une fonction acide carboxylique, et une forme solide, plus particulièrement pulvérulente, et avec une pureté supérieure à 80%.

Le procédé Schotten-Baumann précédemment décrit présente comme avantage de conduire des réactions de N-acylation avec une cinétique rapide, du fait de la réactivité très élevée des chlorures d'acide vis-à-vis des composés et fonctions nucléophiles (par exemple la fonction amine), sans apport important d'énergie comme par exemple l'énergie thermique, dans un milieu solvant majoritairement constitué d'eau, avec des rendements élevés.

Ce procédé comporte en contrepartie comme inconvénients de conduire les réactions en milieux dilués ce qui diminue alors leur productivité ; d'utiliser des chlorures d'acide comme matières premières, obtenus après des réactions faisant intervenir du chlorure de thionyle ou du trichlorure phosphorique comme réactifs, connus pour leur dangerosité ; d'utiliser des co-solvants organiques difficilement recyclables ; et d'induire d'importantes quantités de sels inorganiques, imposant ainsi le traitement des eaux de lavage.

La recherche de procédés de préparation de dérivés N-acylés d'acides aminés, alternatifs à ceux mettant en œuvre la réaction de Schotten Baumann, plus respectueux de l'environnement, constitue donc un problème technique pertinent du domaine technique.

Parmi les solutions techniques, on connait les procédés de préparation de dérivés N-acylés d'acides aminés consistant à mener une réaction d'amidification directe de l'acide aminé par réaction avec un acide gras, ou bien à mener une réaction de trans-amidification de l'acide aminé par réaction avec un ester d'acide gras comme par exemple un ester méthylique ou un ester éthylique.

Le brevet américain publié sous le numéro U.S. 3,836,551, décrit un procédé de préparation d'un dérivé N-acylé d'un acide aminé par la réaction d'un composé portant une fonction acide carboxylique, ou un de ses esters, avec un acide aminé comprenant au moins trois atomes de carbone en présence d'une quantité équimolaire d'une base, à une température comprise entre 100°C et 250°C.

La publication « High temperature reactions of fats with amino acids » (J. Am. Oil Chem. Soc., 1975, 52(5), pp 144 - 147) décrit des réactions menées à haute température (150°C à 200°C), d'esters méthyliques (comme par exemple le myristate de méthyle ou le stéarate de méthyle) avec différents acides aminés. Ces réactions s'accompagnent cependant de réactions de décarboxylation, puis d'amidification, pour conduire à la formation d'amides grasses présentant la chaîne latérale de l'acide aminé, et donc à des structures qui ne sont pas celles recherchées.

La demande de brevet allemand publiée sous le numéro, DE 4 408 957 A1, décrit la préparation de N-acyl sarcosinates à partir d'esters méthyliques, en introduisant une étape intermédiaire de mise en suspension du sarcosinate de sodium avec un agent dispersant dans le mélange de catalyseur, plus particulièrement le méthylate de sodium, et de solvant, plus particulièrement le méthanol.

Le brevet américain publié sous le numéro U.S. 5,710,295, décrit un procédé de préparation de N-acyl aminoacides, par réaction d'un acide gras portant une fonction carboxylique et d'un acide aminé à une température comprise entre 100°C et 200°C, en présence d'un mélange d'eau et de solvant, et plus particulièrement d'eau et d'isopropanol. Cependant l'utilisation d'un solvant comportant une fonction alcool rend probable la formation d'ester comme sous-produit non désiré.

Les demandes internationales publiées sous les numéros WO 2013/014238 et WO 2014 /008103 divulguent des un procédés de préparation de N-acyl aminoacides par réaction entre un acide aminé et un ester d'alkyle, tel qu'un ester méthylique, d'un acide gras comportant de huit à vingt-deux atomes de carbone en présence d'un polyol tel que le glycérol ou le propylèneglycol, notamment en présence d'un oxyde métallique, tel l'oxyde de calcium ou en présence de méthylate de sodium. Un tel procédé permet d'atteindre des produits de réaction faiblement colorés, mais il est mis en œuvre en présence de solvants et d'espèces métalliques que l'on doit éliminer ultérieurement. De plus, la présence de polyols dans le milieu solvant rend probable la formation d'esters non désirés.

La demande de brevet américain publiée sous le numéro US 2006/0239952 décrit un procédé de préparation de N-acyl aminoacides, comme les lauroyl aminoacides, par réaction entre un acide aminé, comme par exemple la glycine, l'alanine ou la thréonine, et une quantité excédentaire d'acide laurique, supérieure notamment à 1,5 équivalents molaire, et en présence d'une substance alcaline, induisant une quantité importante de substances salines. Ce procédé permet d'atteindre des mélanges de dérivés N-acylés d'acides aminés, et notamment d'espèces peptidiques, comme les N-lauroyl gluconate de sodium, N-lauroyl (glycylglycinate) de sodium, et les N-lauroyl (glycylglycylglycinate) de sodium. Les mélanges ainsi atteints, sont utilisés pour la préparation de compositions de nettoyage de la peau humaine.
Le document EP2889306 décrit des compositions cosmétiques à usage topique comprenant un composé de formule (I) : 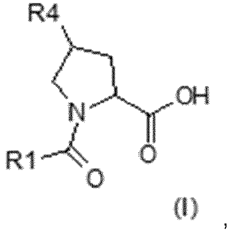 dans laquelle le groupe R₁-C(=O)- représente un radical aliphatique saturé ou insaturé, linéaire ou ramifié, comportant de six à huit atomes de carbone et R₄ représente un atome d'hydrogène ou un radical hydroxyle ;
ainsi qu'un dérivé prolylproline N-acylé et mentionne aussi la préparation des composés de formule (I) par la réaction de Schotten-Baumann utilisant un chlorure d'acide.

La proline est un acide aminé très fréquemment utilisé dans la préparation de dérivés N-acylés car lesdits dérivés N-acylés de la proline présentent plusieurs possibilités d'utilisation, notamment dans les industries de la cosmétique, de la dermocosmétique, de la pharmacie humaine ou vétérinaire, comme par exemple leurs utilisations comme agents solubilisants de molécules hydrophobes dans l'eau, comme par exemple les huiles essentielles.

Un tel art antérieur montrent que les solutions alternatives aux procédés de préparation de dérivés N-acylés d'acides aminés, et notamment aux dérivés N-acylés de la proline, alternatifs à ceux mettant en œuvre la réaction de Schotten-Baumann, mettant en œuvre des acides gras ou des esters d'acides gras d'origine naturelle, ne sont pas satisfaisantes car elles impliquent toujours des catalyseurs notamment d'origine métallique et/ou des solvants organiques, et par conséquent leurs étapes d'élimination et/ou de recyclage, et/ou la mise en œuvre de substances alcalines générant des espèces salines en quantités telles qu'elles nécessitent des étapes de post-traitement.

Un tel art antérieur ne permet pas notamment d'obtenir des dérivés N-acylés de la proline selon un procédé mettant en œuvre des acides gras ou des esters d'acides gras d'origine naturelle, et se caractérisant par un fort taux de conversion des matières premières, et particulièrement par l'obtention d'un faible taux massique résiduel d'acide gras ou d'ester gras, et/ou de proline.

C'est pourquoi, dans le cadre de leurs recherches tendant à faire évoluer le procédé existant vers un procédé mettant en œuvre des matières premières d'origine végétale, dans lequel n'est utilisé aucun solvant organique dangereux, qui conduit à une conversion importante des acides gras, ces acides induisant des irritations de la peau, les inventeurs ont développé le procédé objet de la présente invention.

Selon un premier aspect, l'invention a pour objet un procédé de préparation d'un composé de formule (I) ou d'un mélange de composés de formule (I) : 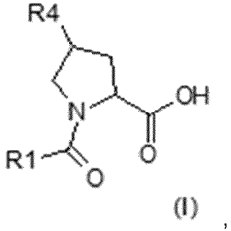 dans laquelle le groupe R₁-C(=O)- représente un radical aliphatique saturé ou insaturé, linéaire ou ramifié, comportant de six à huit atomes de carbone et R₄ représente un atome d'hydrogène ou un radical hydroxyle, ledit procédé comprenant :
- Une **étape a)** dans laquelle un acide gras de formule (II) :

   R₁COOH (II),

   dans laquelle R₁ est tel que défini dans la formule (I), ou un mélange d'acides gras de formule (II), est porté à une température d'au moins 100°C ;
- Une **étape b)** dans laquelle une solution aqueuse de proline ou de 3-hydroxy proline comprenant pour 100% de sa masse, de 30% à 60% massique, plus particulièrement de 40% à 60% massique de proline et de 40% massique à 70% massique, plus particulièrement de 40% à 60% massique d'eau, est versée sur l'acide gras ou le mélange d'acides gras chauffé issu de l'étape a), pour obtenir un mélange (M₁), dans lequel le ratio molaire entre l'acide gras de formule (II) ou le mélange d'acides gras de formule (II) et la proline ou la 3-hydroxy proline, est supérieur ou égal à 0,4/1 et inférieur ou égal à 0,8/1, plus particulièrement supérieur ou égal à 0,5/1 et inférieur ou égal à 0,8/1 et tout particulièrement supérieur ou égal à 0,6/1 et inférieur ou égal à 0,8/1 ;
- Une **étape c)** dans laquelle ledit mélange (M₁) obtenu à l'issue de l'étape b), est maintenu sous agitation mécanique pendant au moins trente minutes et à une température d'au moins 100°C; pour obtenir un mélange aqueux (M₂) du composé de formule (I) telle que définie précédemment et du composé de formule (III) : 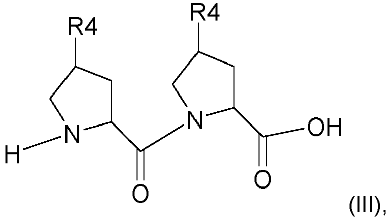
- Une **étape d)** d'isolation dudit composé de formule (I) ou dudit mélange de composés de formule (I), dudit mélange (M₂).

Dans les étapes a) et c) du procédé tel que défini ci-dessus, par au moins 100°C, on signifie que la température peut atteindre 220°C.

Selon un mode particulier du procédé tel que défini précédemment, par au moins 100°C, on désigne dans les étapes a) et c), une température comprise entre 130°C et 220°C, plus particulièrement entre 140°C et 200°C, par exemple entre 140°C et 180°C.

A l'étape c) du procédé tel que défini ci-dessus, par au moins trente minutes, on désigne une durée pouvant atteindre trente heures.

L'étape d) du procédé tel que défini ci-dessus, est généralement réalisée par chromatographie en phase liquide à vocation séparative, par recristallisations successives, par extraction liquide-solide, par extraction liquide-liquide, par extraction utilisant un fluide d'extraction sous sa forme supercritique, l'extraction au dioxyde de carbone à l'état supercritique.

Selon un mode particulier du procédé tel que défini précédemment, par au moins trente minutes, on désigne à l'étape c), une durée comprise entre six heures et vingt heures, plus particulièrement entre huit heures et vingt heures.

Selon un autre mode particulier du procédé tel que défini précédemment, dans la formule (II), le groupe R₁-C(=O)- représente un radical linéaire ou ramifié, saturé ou insaturé, comportant sept ou huit atomes de carbone et tout particulièrement un radical aliphatique linéaire et saturé, comportant sept ou huit atomes de carbone.

Selon un autre mode particulier du procédé tel que défini précédemment, est mise en œuvre à l'étape b), une solution de proline.

Selon un autre mode particulier du procédé tel que défini précédemment, le ratio molaire entre l'acide gras de formule (II) ou le mélange d'acides gras de formule (II) et la proline est supérieur ou égal à 0,6/1 et inférieur ou égal à 0,8/1.

Selon un autre mode particulier du procédé tel que défini ci-dessus, celui-ci comprend une étape e) neutralisation, et/ou de filtration et et/ou de décoloration.

L'invention a aussi pour objet le mélange (M₂), intermédiaire du procédé tel que défini précédemment.

L'invention a également pour objet une composition à usage topique (C) comprenant pour 100% de sa masse :
- De 0,1% à 40% massique, plus particulièrement de 0,1% à 20% massique, et encore plus particulièrement de 0,1% à 5% massique du mélange (M₂) telle que défini ci-dessus, et
- De 60% à 99,9%, plus particulièrement de 80% à 99,9%, et encore plus particulièrement de 95% à 99,9% massique d'un milieu cosmétiquement acceptable.

L'expression "à usage topique" utilisée dans la définition de la composition cosmétique, dermocosmétique, dermopharmaceutique ou pharmaceutique à usage topique (C) telle que définie ci-dessus, signifie que ladite composition est mise en œuvre par application sur la peau, les cheveux, le cuir chevelu ou les muqueuses, qu'il s'agisse d'une application directe dans le cas d'une composition cosmétique, dermocosmétique, dermopharmaceutique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

L'expression "cosmétiquement acceptable" utilisée dans la définition de la composition cosmétique, dermocosmétique, dermopharmaceutique ou pharmaceutique à usage topique (C), signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état. Un milieu cosmétiquement acceptable de ces compositions objet de l'invention peut contenir classiquement de l'eau, un ou plusieurs solvants organiques cosmétiquement acceptables, un mélange d'eau et d'un ou plusieurs solvants organiques. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, l'hexylène glycol, le diéthylène glycol, le xylitol, l'érythritol, le sorbitol, ou les alcools hydrosolubles.

La composition cosmétique, dermocosmétique, dermopharmaceutique ou pharmaceutique à usage topique (C) objet de la présente invention peut être conditionnée sous forme pressurisée dans un dispositif aérosol ou dans un dispositif de type « flacon pompe », dans un dispositif muni d'une paroi ajourée par exemple une grille, dans un dispositif muni d'un applicateur a billes (dit"roll-on). Lorsqu'elle est conditionnée dans des flacons, la composition (C) selon l'invention telle que définie précédemment peut être appliquée sous la forme de fines gouttelettes au moyen de dispositifs de pressurisation mécanique ou à gaz propulseur. Parmi les agents propulseurs que l'on peut associer à la composition (C) selon l'invention, il y a les composés hydrofluorés, comme le dichlorodifluorométhane, le trichlorofluorométhane, le difluoréthane, l'isobutane, le butane, le propane.

La composition cosmétique, dermocosmétique, dermopharmaceutique ou pharmaceutique à usage topique (C) telle que définie précédemment peut en outre comporter des excipients et/ou des principes actifs habituellement mis en œuvre dans le domaine des formulations à usage topique, en particulier cosmétiques, dermocosmétiques, pharmaceutiques ou dermo-pharmaceutique.

La composition cosmétique, dermocosmétique, dermopharmaceutique ou pharmaceutique à usage topique (C) objet de la présente invention et telle que définie précédemment, peut comprendre en outre, un ou plusieurs composés auxiliaires choisis parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents opacificants, les agents nacrants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Parmi les agents antioxydants hydrosolubles que l'on peut associer à la composition cosmétique, dermocosmétique, dermopharmaceutique ou pharmaceutique à usage topique (C) selon l'invention, il y a l'acide ascorbique, la glutathione, l'acide tartrique, l'acide oxalique, le tétra sodium glutamate diacétate.

Parmi les agents séquestrants hydrosolubles que l'on peut associer à la composition cosmétique, dermocosmétique, dermopharmaceutique ou pharmaceutique à usage topique (C) selon l'invention, il y a les sels de l'acide éthylènediamine tétracétique (EDTA), comme le sel de sodium de l'EDTA, les sels de l'acide diéthylènetriamine pentacétique (DTPA) comme les sels de sodium du DTPA, l'acétyl glutamique acide (gamme Dissolvine).

Parmi les colorants hydrosolubles que l'on peut associer à la composition cosmétique, dermocosmétique, dermopharmaceutique ou pharmaceutique à usage topique (C) selon l'invention, il y a le caramel, le Yellow 5, l'Acid Blue 9/ Blue 1, le Green 5, le Green 3 / Fast Green FCF 3, l'Orange 4, le Red 4 / Food Red 1, le Yellow 6, l'Acid Red 33 / Food Red 12, le Red 40, le carmine de cochenille (Cl 15850, Cl 75470), l'Ext. Violet 2, le Red 6-7, le Ferric Ferrocyanide, les Ultramarines, l'Acide Yellow 3 / Yellow 10, l'Acid Blue 3, le Yellow 10.

Parmi les agents hydrosolubles stabilisant de la couleur que l'on peut associer à la composition cosmétique, dermocosmétique, dermopharmaceutique ou pharmaceutique à usage topique (C) selon l'invention, il y a le citrate de Tris(tétraméthyl hydroxy pipéridinol), benzotriazolyl butylphénol sulfonate de sodium, le benzotriazolyl dodecyl p-crésol.

Comme exemples de tensioactifs moussants et/ou détergents, éventuellement présents dans la composition cosmétique, dermocosmétique, dermopharmaceutique ou pharmaceutique à usage topique (C) objet de la présente invention, on peut citer les tensioactifs moussants et/ou détergents anioniques, cationiques, amphotères ou non ioniques topiquement acceptables habituellement utilisés dans ce domaine d'activité. Parmi les tensioactifs anioniques moussants et/ou détergents que l'on peut associer à la composition cosmétique, dermocosmétique, dermopharmaceutique ou pharmaceutique à usage topique (C) objet de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras. Parmi les tensioactifs amphotères moussants et/ou détergents éventuellement présents dans la composition cosmétique, dermocosmétique, dermopharmaceutique ou pharmaceutique à usage topique (C) objet de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates. Parmi les tensioactifs cationiques moussants et/ou détergents éventuellement présents dans la composition cosmétique, dermocosmétique, dermopharmaceutique ou pharmaceutique à usage topique (C) objet de la présente invention, on peut citer particulièrement les dérivés d'ammoniums quaternaires.

Parmi les tensioactifs non ioniques moussants et/ou détergents éventuellement présents dans la composition cosmétique, dermocosmétique, dermopharmaceutique ou pharmaceutique à usage topique (C) objet de la présente invention, on peut citer plus particulièrement les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah, les N-alkylamines. Comme exemples d'agents de texture éventuellement présents dans la composition cosmétique, dermocosmétique, dermopharmaceutique ou pharmaceutique à usage topique (C) objet de la présente invention, on peut citer des dérivés N-acylés d'acides aminés, par exemple la lauroyl lysine commercialisée sous l'appellation AMlNOHOPE™LL, l'octenyl starch succinate commercialisé sous l'appellation DRYFLO™, le myristyl polyglucoside commercialisé sous l'appellation MONTANOV 14, les fibres de cellulose, les fibres de coton, les fibres de chitosane, le talc, la séricite, le mica, la perlite.

Comme exemples de principes actifs éventuellement présents dans la composition cosmétique, dermocosmétique, dermopharmaceutique ou pharmaceutique à usage topique (C) objet de la présente invention, il y a :
- Les vitamines et leurs dérivés, par exemple, le rétinol (vitamine A) et ses esters (palmitate de rétinyle par exemple), l'acide ascorbique (vitamine C) sous forme de sel et ses esters, les dérives de sucre de l'acide ascorbique (par exemple l'ascorbyl glucoside), le tocophérol (vitamine E) et ses esters (par exemple l'acétate de tocophérol), les vitamines B3 ou B10 (niacinamide et ses dérivés) ;
- Les composés ayant une action éclaircissante ou dépigmentante de la peau, par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™ ;
- Les composés ayant une action apaisante comme le SEPICALM™ S, l'allantoïne et le bisabolol ;
- Les agents anti-inflammatoires ;
- Les composés montrant une action hydratante par exemple le diglycérol, le triglycérol, l'urée, les hydroxy urées, le glycérol glucoside, le diglycérol glucoside, les polyglycéryl glucosides, l'érythrityl glucoside, le sorbityl glucoside, le xylityl glucoside, la composition commercialisée sous le nom de marque AQUAXYL™ comprenant du xylityl glucoside, de l'anhydroxylitol et du xyllitol ;
- Les composés montrant une action amincissante ou lipolytique comme la caféine ou ses dérivés, l'ADIPOSLIM™, l'ADIPOLESS™ ;
- Les extraits végétaux riches en tanins en polyphénols et/ ou en isoflavones, par exemple les extraits de raisin, les extraits de pin, les extraits de vin, les extraits d'olives ; les extraits de soja, par exemple la Raffermine™ ; les extraits de blé par exemple la TENSINE™ ou la GLIADINE™ ; les extraits végétaux riches en terpènes ; les extraits d'algues d'eau douce ou marines ; les extraits marins en général comme les coraux ;
- Les composés ayant une action antimicrobienne ou une action purifiante, par exemple le LIPACIDE™C8G, le LIPACIDE™UG, le SEPICONTROL™ A5 ; l'OCTOPIROX™ ou le SENSIVA™SC50 ;
- Les composés ayant une propriété énergisante ou stimulante comme le Physiogényl™, le panthénol et ses dérivés comme le SEPICAP™MP ;
- Les actifs anti-âge comme le SEPILIFT™DPHP, le LIPACIDE™PVB, le SEPIVINOL™, le SEPIVITAL™, le MANOLIVA™, le PHYTO-AGE™, le TIMECODE™ ; le SURVICODE™ ;
- Les actifs anti-photovieillissement;
- Les actifs augmentant la synthèse des composants de la matrice extracellulaire par exemple le collagène, les élastines, les glycosaminoglycanes ;
- Les actifs agissant favorablement sur la communication cellulaire chimique comme les cytokines ou physiques comme les intégrines ;
- Les actifs créant une sensation de « chauffe » sur la peau comme les activateurs de la microcirculation cutanée (par exemple les dérivés de l'acide nicotinique) ou des produits créant une sensation de « fraîcheur » sur la peau (par exemple le menthol et des dérivés) ;
- Les actifs améliorant la microcirculation cutanée, par exemple les veinotoniques ; les actifs drainants ; les actifs à visée décongestionnante par exemple les extraits de ginko biloba, de lierre, de marron d'inde, de bambou, de ruscus, de petit houx, de centalla asiatica, de fucus, de romarin, de saule ;
- Les actifs agissant comme agents tenseurs de la peau, par exemple les hydrolysats de protéines végétales, les hydrolysats d'origine marine comme les hydrolysats d'extraits de laminaires, les hydrolysats de cartilages de poissons, l'élastine marine, le produit commercialisé par la société SEPPIC sous le nom de marque SESAFLASH™, les solutions de collagène.
- Les agents de bronzage ou de brunissement de la peau, par exemple la dihydroxyacétone, l'isatine, l'alloxane, la ninhydrine, le glycéraldéhyde, l'aldéhyde mésotartrique, la glutaraldéhyde, l'érythrulose.

Comme exemples d'agents déodorants éventuellement présents dans la composition cosmétique, dermocosmétique, dermopharmaceutique ou pharmaceutique à usage topique (C) objet de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol, le caprylate de glycérol, le caprate de polyglycérol, le 1,2-décanediol, le 1,3-propanediol, l'acide salicylique, le bicarbonate de sodium, les cyclodextrines, les zéolithes métalliques, le Triclosan™, le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'agents épaississants ou gélifiants éventuellement présents dans la composition cosmétique, dermocosmétique, dermopharmaceutique ou pharmaceutique à usage topique (C) objet de la présente invention, on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- méthyl)acrylamido méthane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymères linéaire, branché ou réticulé d'au moins un monomère possédant une fonction acide fort, libre, partiellement salifiée ou totalement salifiée, avec au moins un monomère neutre, et au moins un monomère de formule (VIII):

CH₂=C(R'₃)-C(=O)-[CH₂-CH₂-O]ₙ-R'₄ (VIII)

dans laquelle R'₃ représente un atome d'hydrogène ou un radical méthyle, R'₄ représente un radical alkyle linéaire ou ramifié comportant de huit à trente atomes de carbone et n représente un nombre supérieur ou égal à un et inférieur ou égal à cinquante;

Les polymères de type polyélectrolytes, linéaires ou branchés ou réticulés que l'on peut associer à la composition cosmétique, dermocosmétique, dermopharmaceutique ou pharmaceutique à usage topique (C) objet de la présente invention, peuvent se présenter sous la forme d'une solution, d'une suspension aqueuse, d'une émulsion eau-dans-huile, d'une émulsion huile-dans-eau, d'une poudre, par exemple les produits commercialisés sous les appellations SIMULGEL™ EG, SIMULGEL™EPG, SEPIGEL™ 305, SIMULGEL™ 600, SIMULGEL™ NS, SIMULGEL™ INS 100, SIMULGEL™ FL, SIMULGEL™ A, SIMULGEL™ SMS 88, SEPINOV™EMT 10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC 2, ARISTOFLEX™HMB, COSMEDIA™SP, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100; les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ); les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes; la cellulose, les dérivés de cellulose comme la méthylcellulose, l'éthyl-cellulose, l'hydroxypropyl cellulose, les silicates, l'amidon, les dérivés hydrophiles de l'amidon, les polyuréthanes.

Comme exemples d'huiles éventuellement présentes dans la composition cosmétique, dermocosmétique, dermopharmaceutique ou pharmaceutique à usage topique (C) objet de la pré sente invention, on peut citer les huiles minérales telles que l'huile de paraffine, l'huile de vaseline, les isoparaffines ou les huiles blanches minérales ; les huiles d'origine animale, telles que le squalène ou le squalane ;les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de graines de coriandre, l'huile de faines, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires éventuellement présentes dans la composition cosmétique, dermocosmétique, dermopharmaceutique ou pharmaceutique à usage topique (C) objet de la pré sente invention, on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de tensioactifs non-ioniques émulsionnants que l'on peut associer à la composition cosmétique, dermocosmétique, dermopharmaceutique ou pharmaceutique à usage topique (C) objet de la présente invention, on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre cinq moles et cent cinquante moles d'oxyde d'éthylène, comme la composition comprenant de l'acide stéarique éthoxylé à cent trente-cinq moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters de méthyl glucoside ; les alkyl polyglycosides comportant un radical aliphatique, linéaire ou ramifié, saturé ou insaturé, et comportant de quatorze à trente-six atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le (2-octyl dodécyl) polyxyloside, le (12-hydroxy stéaryl) polyglucoside ; les compositions d'alcools gras linéaires ou ramifiés, saturés ou insaturés, et comportant de quatorze à trente-six atomes de carbone, et d'alkyl polyglycosides tels que décrits précédemment, par exemple les compositions commercialisées sous les noms de marque MONTANOV™68, MONTANOV™14, MONTANOV™82, MONTANOV™202, MONTANOV™S, MONTANOV™WO18, MONTANOV™L, FLUIDANOV™20X et EASYNOV™.

Comme exemples d'agent de protection contre les rayonnements ultra-violets du soleil éventuellement présents dans la composition cosmétique, dermocosmétique, dermopharmaceutique ou pharmaceutique à usage topique (C) objet de la présente invention, on désigne les pigments, les filtres organiques solaires et les filtres inorganiques solaires.

Comme pigments utilisés comme agent de protection contre les rayonnements ultra-violets du soleil éventuellement présents dans la composition cosmétique, dermocosmétique, dermopharmaceutique ou pharmaceutique à usage topique (C) objet de la présente invention, il y a par exemple le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Comme filtres organiques solaires utilisés comme agent de protection contre les rayonnements ultra-violets du soleil éventuellement présents dans la composition cosmétique, dermocosmétique, dermopharmaceutique ou pharmaceutique à usage topique (C) objet de la présente invention, il y a par exemple:
- Ceux de la famille des dérivés de l'acide benzoïque comme les acides paraaminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Ceux de la famille des dérivés de l'acide anthranilique comme l'homomenthyl-N-acétyl anthranilate;
- Ceux de la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle;
- Ceux de la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-□α-cyano-β-phényle, le cinnamate de diparaméthoxy mono-2-éthylhexanoyl de glycéryle;
- Ceux de la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 2-éthylhexyl 4'-phényl benzophénone-2-carboxylate, la 2-hydroxy 4-n-octyloxy benzophénone, la 4-hydroxy 3-carboxy benzophénone ; le 3-(4'-méthylbenzylidène) d,l-camphre, le 3-(benzylidène)-d,l-camphre, le benzalkonium méthosulfate camphre ; l'acide urocanique, l'urocanate d'éthyle ;
- Ceux de la famille des dérivés de l'acide sulfonique comme l'acide 2-phényl benzimidazole-5-sulfonique et ses sels; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy) 1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthyl benzoxazole, le 2,2'-hydroxy-5-méthylphényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbornylidène)-3-pentan-2-one ; le 2-(4-diethylamino 2-hydroxy benzoyl) benzoic acid hexyl ester, le 2,4-bis{[4-(2-ethylhexyloxy)-2-hydroxy] phenyl}-6-(4-methoxy phenyl) 1,3,5-triazine, le 2,4,6-tris[4-(2-ethylhexyloxycarbonyl) anilino]-1,3,5-triazine, le 2-ethylhexyl dimethoxybenzylidene dioxoimidazolidine propionate, la famille des dérivés du diphénylacrylate comme le 2-éthylhexyl-2-cyano-3,3-diphényl-2-propènoate, l'éthyl-2-cyano-3,3-diphényl-2-propènoate;
- Ceux de la famille des polysiloxanes comme le malonate de benzylidène siloxane.

Comme filtres inorganiques solaires utilisés comme agent de protection contre les rayonnements ultra-violets du soleil éventuellement présents dans la composition cosmétique, dermocosmétique, dermopharmaceutique ou pharmaceutique à usage topique (C) objet de la présente invention, il y a par exemple: les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Les exemples suivants illustrent l'invention sans toutefois la limiter.

### Exemple 1 : préparation d'une Composition (C_{1A}) selon l'invention

On introduit 62,6 grammes d'acide n-heptanoïque, soit 0,8 équivalent molaire, dans un réacteur muni d'une double enveloppe comprenant un fluide caloporteur, d'un système d'agitation mécanique équipé d'une pâle d'agitation de type hélice ou ancre, d'un système d'évacuation d'eau. La température est portée à 150°C et l'agitation est mise en marche pour homogénéiser l'acide heptanoïque pendant une période de 10 minutes.

On ajoute ensuite progressivement 69,54 grammes d'une solution aqueuse de proline, contenant 50% massique de proline soit un équivalent molaire de proline, et on maintien un tel mélange pendant une durée de douze heures et trente minutes, à une température de 155°C, sous un vide partiel de 4x10⁴Pa à 7x10⁴Pa (400 à 700mbar). Le milieu réactionnel obtenu est refroidi à une température de 90°C, puis vidangé pour obtenir la composition (C_{1A}), qui est ensuite caractérisée analytiquement.

### Exemple 2 : préparation d'une Composition (C_{1B}) selon l'invention

Le mode opératoire du procédé décrit dans l'exemple 1 est mis en œuvre en remplaçant le 0,8 équivalent molaire d'acide n-heptanoïque par 0,8 équivalent molaire d'acide n-octanoïque.

Un tel mélange est alors maintenu pendant une durée de onze heures, à une température de 155°C, sous un vide partiel de 2x10⁴Pa à 4x10⁴Pa (200 à 400mbar). Le milieu réactionnel obtenu est refroidi à une température de 90°C, puis vidangé pour obtenir la composition (C_{1B}), qui est ensuite caractérisée analytiquement.

### Exemple Comparatif 1 : préparation d'une Composition (C'₁) comparative

Le mode opératoire du procédé décrit dans l'exemple 1 est mis en œuvre en remplaçant le 0,8 équivalent molaire d'acide n-heptanoïque par 0,8 équivalent molaire d'acide n-laurique.

Un tel mélange est alors maintenu pendant une durée de six heures et trente minutes, à une température de 155°C, sous un vide partiel de 3x10⁴Pa (300mbar). Le milieu réactionnel obtenu est refroidi à une température de 90°C, puis vidangé pour obtenir la composition comparative (C'₁), qui est ensuite caractérisée analytiquement.

### Exemple Comparatif 2 : préparation d'une Composition (C'₂) comparative

Le mode opératoire du procédé décrit dans l'exemple 1 est mis en œuvre en remplaçant le 0,8 équivalent molaire d'acide n-heptanoïque par 0,8 équivalent molaire d'acide n-palmitique.

Un tel mélange est alors maintenu pendant une durée de six heures et trente minutes, à une température de 155°C, sous un vide partiel de 2x10⁴Pa à 6x10⁴Pa (200 à 600mbar). Le milieu réactionnel obtenu est refroidi à une température de 90°C, puis vidangé pour obtenir la composition comparative (C'₂), qui est ensuite caractérisée analytiquement.

### Exemple Comparatif 3 : préparation d'une Composition (C'₃) comparative

Le mode opératoire du procédé décrit dans l'exemple 1 est mis en œuvre en remplaçant le 0,8 équivalent molaire d'acide n-heptanoïque par 0,8 équivalent molaire d'acide n-octanoïque, et le un équivalent molaire de proline par un équivalent molaire de glycine.

Aucune réaction n'est observée et le milieu prend en masse. La composition (C'₃) comparative consiste donc en un mélange d'acide octanoïque et de glycine.

### Caractérisation des compositions (C_{1A}) et (C_{1B}) selon l'invention, et des compositions comparatives, (C'₁), (C'₂) et (C'₃).

Les compositions (C_{1A}) et (C_{1B}) selon l'invention, et les compositions comparatives, (C'₁), (C'₂) et (C'₃) obtenues, sont caractérisées par l'évaluation visuelle de l'aspect, la mesure de l'Indice d'acide ainsi que la détermination des teneurs massiques en acide gras résiduel, proline résiduelle, diproline, dérivé N-acylé de la proline et dérivé N-acylé dipeptidique de la proline par chromatographie en phase gazeuse au moyen d'un chromatographe comprenant un système d'injection, un détecteur d'ionisation de flamme muni d'un système d'acquisition de données et équipé d'une colonne CP SIMDIST ULTIMETAL, (100% méthylsiloxane 10 m x 0,53 mm ID, épaisseur de film 0,53 µm) en utilisant l'hélium comme gaz vecteur.

La conversion des matières premières sera jugée insatisfaisante, si la teneur massique en acide gras dans la composition issue du procédé d'obtention de dérivés N-acylés de la proline, est supérieure ou égale à 10% pour 100% massique de la composition obtenue. En effet, au-delà d'une telle teneur massique, des réactions d'irritation de la peau, causées par l'acide gras, sont susceptibles d'être rencontrées et de rendre alors impropre à un usage cosmétique une telle composition.

Les caractérisations analytiques des compositions (C_{1A}) et (C_{1B}) selon l'invention, et les compositions comparatives, (C'₁), (C'₂) et (C'₃) obtenues, sont consignées dans les tableaux 1 et 2 suivants :

**Tableau 1**

| | Composition (C_{1A}) | Composition (C_{1B}) |
|---|---|---|
| Aspect à 25°C (visuel) | Liquide visqueux | Liquide visqueux |
| Indice d'acide (mg KOH/g) (NFT 60-204) | 142,8 | 124,0 |
| Acide heptanoïque (% massique) | 2,0% | - |
| Acide octanoïque (% massique) | - | 1,4% |
| Proline (% massique) | n.d. | 0,4% |
| Proline-Proline (% massique) | 28,3% | 22,4% |
| N-heptanoyl Proline(% massique) | 67,5% | - |
| N-octanoyl Proline (% massique) | - | 55,6% |
| N-heptanoyl Proline - Proline (% massique) | 2,2% | - |
| N-octanoyl Proline - Proline (% massique) | - | 9,2% |

| | | |
|---|---|---|
| n.d. : non déterminé. | | |

**Tableau 2**

| | Composition (C'₁) | Composition (C'2) |
|---|---|---|
| Aspect à 25°C (visuel) | Liquide visqueux | Liquide visqueux |
| Indice d'acide (mg KOH/g) (NFT 60-204) | 164,0 | 145,0 |
| Acide laurique (% massique) | 30,4% | - |
| Acide palmitique (% massique) | - | 45,2% |
| Proline (% massique) | n.d. | n.d. |
| Proline-Proline (% massique) | 9,7 % | 6,8% |
| N-lauroyl Proline (% massique) | 57,6% | - |
| N-palmitoyl Proline (% massique) | - | 46,7% |
| N-lauroyl Proline - Proline (% massique) | 2,3% | - |
| N-palmitoyl Proline - Proline (% massique) | - | 1,3 % |

Ces caractérisations mettent en évidence que les teneurs massiques des acides gras résiduels et des composés de formules (II) des compositions (C1_{A}) et (C1_{B}) obtenues par la mise en œuvre du procédé selon l'invention, sont nettement inférieur à 10% massique alors que celles en acides gras des compositions comparatives (C'₁) et (C'₂) sont au contraire nettement supérieures à 10% massique.

## Revendications

1. Procédé de préparation d'un composé de formule (I) ou d'un mélange de composés de formule (I) : 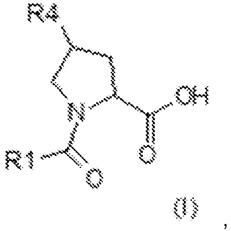 dans laquelle le groupe R₁-C(=O)- représente un radical aliphatique saturé ou insaturé, linéaire ou ramifié, comportant de six à huit atomes de carbone et R₄ représente un atome d'hydrogène ou un radical hydroxyle, ledit procédé comprenant :
- Une **étape a)** dans laquelle un acide gras de formule (II) :
R₁COOH (II),
dans laquelle R₁ est tel que défini dans la formule (I), ou un mélange d'acides gras de formule (II), est porté à une température d'au moins 100°C ;
- Une **étape b)** dans laquelle une solution aqueuse de proline ou de 3-hydroxy proline comprenant pour 100% de sa masse de 30% à 60% massique de proline et de 40% massique à 70% massique d'eau, est versée sur l'acide gras ou le mélange d'acides gras chauffé issu de l'étape a), pour obtenir un mélange (M₁), dans lequel le ratio molaire entre l'acide gras de formule (II) ou le mélange d'acides gras de formule (II) et la proline ou la 3-hydroxy proline, est supérieur ou égal à 0,4/1 et inférieur ou égal à 0,8/1 ;
- Une **étape c)** dans laquelle ledit mélange (M₁) obtenu à l'issue de l'étape b), est maintenu sous agitation mécanique pendant au moins trente minutes et à une température d'au moins 100°C; pour obtenir un mélange aqueux (M₂) du composé de formule (I) telle que définie précédemment et du composé de formule (III) : 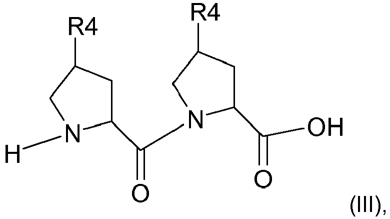
- Une **étape d)** d'isolation dudit composé de formule (I) ou dudit mélange de composés de formule (I), dudit mélange (M₂).

2. Procédé tel que défini à la revendication 1 **caractérisé en ce que** dans la formule (I), le groupe R₁-C(=O)- représente un radical n-heptanoyle ou un radical n-octanoyle.

3. Procédé tel que défini à l'une des revendications 1 ou 2, **caractérisé en ce que** à l'étape b), est mise en œuvre une solution de proline.

4. Procédé tel que défini à la revendication 3, **caractérisé en ce que** le ratio molaire entre l'acide gras de formule (II) ou le mélange d'acides gras de formule (II) et la proline est supérieur ou égal à 0,6/1 et inférieur ou égal à 0,8/1.

5. Mélange (M₂), intermédiaire du procédé tel que défini à la revendication 1.

6. Mélange (M₂), intermédiaire du procédé tel que défini à la revendication 2.

7. Mélange (M₂), intermédiaire du procédé tel que défini à la revendication 3.

8. Mélange (M₂), intermédiaire du procédé tel que défini à la revendication 4.

9. Composition cosmétique, dermocosmétique, dermopharmaceutique ou pharmaceutique à usage topique (C) comprenant pour 100% de sa masse :
- De 0,1% à 40% massique du mélange (M₂) telle que défini à l'une ou quelconque des revendications 5 à 8, et
- De 60% à 99,9% massique d'un milieu cosmétiquement acceptable.

## Patentansprüche

1. Verfahren zur Herstellung einer Verbindung der Formel (I) oder eines Gemischs von Verbindungen der Formel (I): 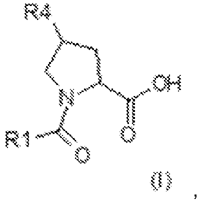 in der die Gruppe R₁-C(=O)- für einen gesättigten oder ungesättigten, linearen oder verzweigten aliphatischen Rest mit sechs bis acht Kohlenstoffatomen steht und R₄ für ein Wasserstoffatom oder einen Hydroxylrest steht, wobei das Verfahren Folgendes umfasst:
- einen **Schritt a),** in dem man eine Fettsäure der Formel (II):
R₁COOH (II),
in der R₁ wie in Formel (I) definiert ist, oder ein Gemisch von Fettsäuren der Formel (II) auf eine Temperatur von mindestens 100 °C bringt;
- einen **Schritt b),** in dem man eine wässrige Lösung von Prolin oder 3-Hydroxyprolin, die pro 100 Gew.-% ihrer Masse 30 bis 60 Massen-% Prolin und 40 Massen-% bis 70 Massen-% Wasser umfasst, auf die erhitzte Fettsäure bzw. das erhitzte Gemisch von Fettsäuren aus Schritt a) gießt, was ein Gemisch (M₁) ergibt, in dem das Molverhältnis von Fettsäure der Formel (II) bzw. Gemisch von Fettsäuren der Formel (II) zu Prolin bzw. 3-Hydroxyprolin größer oder gleich 0,4/1 und kleiner oder gleich 0,8/1 ist;
- einen **Schritt c),** in dem man das am Ende von Schritt b) erhaltenen Gemisch (M₁) unter mechanischem Rühren über einen Zeitraum von mindestens dreißig Minuten und bei einer Temperatur von mindestens 100 °C unter mechanischem Rühren hält, was ein wässriges Gemisch (M₂) der Verbindung der Formel (I) gemäß obiger Definition und der Verbindung der Formel (III): 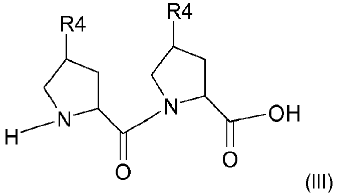 ergibt;
- einen **Schritt d)** der Isolierung der Verbindung der Formel (I) bzw. des Gemischs von Verbindungen der Formel (I) aus dem Gemisch (M₂),

2. Verfahren nach Anspruch 1, **dadurch gekennzeichnet, dass** in Formel (I) die Gruppe R₁-C(=O)- für einen n-Heptanoylrest oder einen n-Octanoylrest steht.

3. Verfahren nach Anspruch 1 oder 2, **dadurch gekennzeichnet, dass** in Schritt b) eine Lösung von Prolin eingesetzt wird.

4. Verfahren nach Anspruch 3, **dadurch gekennzeichnet, dass** das Molverhältnis von Fettsäure der Formel (II) bzw. Gemisch von Fettsäuren der Formel (II) zu Prolin größer oder gleich 0,6/1 und kleiner oder gleich 0,8/1 ist.

5. Gemisch (M₂), bei dem es sich um ein Zwischenprodukt des Verfahrens nach Anspruch 1 handelt.

6. Gemisch (M₂), bei dem es sich um ein Zwischenprodukt des Verfahrens nach Anspruch 2 handelt.

7. Gemisch (M₂), bei dem es sich um ein Zwischenprodukt des Verfahrens nach Anspruch 3 handelt.

8. Gemisch (M₂), bei dem es sich um ein Zwischenprodukt des Verfahrens nach Anspruch 4 handelt.

9. Kosmetische, dermokosmetische, dermopharmazeutische oder pharmazeutische Zusammensetzung zur topischen Verwendung (C), umfassend pro 100 % ihrer Masse:
- 0,1 bis 40 Massen-% des Gemischs (M₂) nach einem der Ansprüche 5 bis 8 und
- 60 bis 99,9 Massen-% eines kosmetisch unbedenklichen Mediums.

## Claims

1. Process for the preparation of a compound of formula (I) or of a mixture of compounds of formula (I): 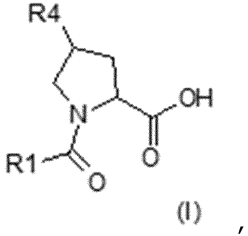 in which the R₁-C(=O)- group represents a saturated or unsaturated and linear or branched aliphatic radical comprising from 6 to 8 carbon atoms and R₄ represents a hydrogen atom or a hydroxyl radical, said process comprising:
- a **stage a)** in which a fatty acid of formula (II):
R₁COOH (II),
in which R₁ is as defined in the formula (I), or a mixture of fatty acids of formula (II), is brought to a temperature of at least 100°C;
- a **stage b)** in which an aqueous solution of proline or of 3-hydroxyproline comprising, per 100% of its weight, from 30% to 60% by weight of proline and from 40% to 70% by weight of water is poured onto the heated fatty acid or the heated mixture of fatty acids resulting from stage a), in order to obtain a mixture (M₁) in which the molar ratio of the fatty acid of formula (II) or the mixture of fatty acids of formula (II) to proline or 3-hydroxyproline is greater than or equal to 0.4/1 and less than or equal to 0.8/1;
- a **stage c)** in which said mixture (M₁) obtained on conclusion of stage b) is maintained under mechanical stirring for at least 30 minutes and at a temperature of at least 100°C, in order to obtain an aqueous mixture (M₂) of the compound of formula (I) as defined above and of the compound of formula (III): 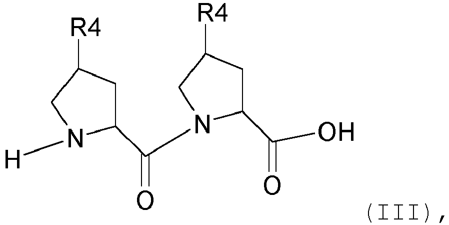
- a **stage d)** of isolation of said compound of formula (I) or of said mixture of compounds of formula (I) from said mixture (M₂).

2. Process as defined in Claim 1, **characterized in that**, in the formula (I), the R₁-C(=O)- group represents an n-heptanoyl radical or an n-octanoyl radical.

3. Process as defined in either of Claims 1 and 2, **characterized in that**, in stage b), a proline solution is employed.

4. Process as defined in Claim 3, **characterized in that** the molar ratio of the fatty acid of formula (II) or the mixture of fatty acids of formula (II) to proline is greater than or equal to 0.6/1 and less than or equal to 0.8/1.

5. Mixture (M₂), which is an intermediate of the process as defined in Claim 1.

6. Mixture (M₂), which is an intermediate of the process as defined in Claim 2.

7. Mixture (M₂), which is an intermediate of the process as defined in Claim 3.

8. Mixture (M₂), which is an intermediate of the process as defined in Claim 4.

9. Cosmetic, dermocosmetic, dermopharmaceutical or pharmaceutical composition for topical use (C) comprising, per 100% of its weight:
- from 0.1% to 40% by weight of the mixture (M₂) as defined in any one of Claims 5 to 8, and
- from 60% to 99.9% by weight of a cosmetically acceptable medium.
